# EUROPÄISCHE PATENTANMELDUNG

(11) **EP 2 543 359 A1**
(43) Veröffentlichungstag der Anmeldung: **09.01.2013**
(21) Anmeldenummer: 11005618.1
(22) Anmeldetag: 08.07.2011
(51) Int. Cl.: A61K 9/06, A61K 33/20, A61K 47/02

(54) **Gel zur Verwendung in einem Wundbehandlungsmittel**

(71) Anmelder: Caliopa AG, 6314 Unterägeri (CH)
(72) Erfinder: Mathé, Hans-Georg, 6330 Cham (CH)
(74) Vertreter: Walkenhorst, Andreas

(57) **Zusammenfassung**

Es soll ein Gel, insbesondere zur Verwendung in einem Wundbehandlungsmittel, angegeben werden, mit dem bei hoher mikrobiozider Wirksamkeit eine besonders hohe Lagerbeständigkeit und Langzeitstabilität erreichbar ist. Dazu umfasst das Gel erfindungsgemäß ein anorganisches Silikat als Verdicker und eine elektrochemisch aktivierte wässrige Kochsalzlösung, wobei die elektrochemisch aktivierte wässrige Kochsalzlösung erhältlich ist durch Vermischen von elektrochemisch aktivierter Ausgangs-Kochsalzlösung, die einen Gehalt an freiem Chlor von mehr als 300 mg/l aufweist, mit destilliertem Wasser, und wobei die elektrochemisch aktivierte wässrige Kochsalzlösung eine Leitfähigkeit von höchstens 12 mS/cm aufweist.

## Beschreibung

Die Erfindung bezieht sich auf ein Gel, insbesondere zur Verwendung in einem Wundbehandlungsmittel, auf Schleimhäuten, stark durchbluteten Hautregionen, beispielsweise auch Sexualorganen, oder anderen Anwendungen für den humanen oder tierischen Gebrauch.

In der nicht vorveröffentlichten EP 10 003 555.9 ist ein auch als "Aseca-Verfahren" bezeichnetes Verfahren zur Herstellung einer elektrochemisch aktivierten Salz-Lösung, auch bezeichnet als Anolyt oder "Aseca-Lösung", beschrieben.

Die Offenbarung der EP 10 003 555.9 wird bezüglich des Herstellungsverfahrens, der hierfür vorgesehenen Medienstromführung, der vorgesehenen Betriebsparameter, der zur Herstellung verwendeten Anlage sowie der genannten Stoffeigenschaften ausdrücklich miteinbezogen ("incorporation by reference").

Die mit dem in der EP 10 003 555.9 beschriebenen Verfahren hergestellte elektrochemisch aktivierte Salzlösung (Anolyt, "Aseca-Lösung") ist ein hochwirksames Desinfektionsmittel mit außergewöhnlichen mikrobioziden Eigenschaften, das insbesondere durch Elektrolyse von Natriumchlorid-Lösungen gewonnen werden kann. Derartiger Anolyt kann beispielsweise, je nach gewählter Verdünnung, in Anwendungen zur Flächendesinfektion eingesetzt werden, z. B. von Arbeitsplatten, Tischen, Böden, aber auch für Kaltdesinfizierungshandlungen, in der Landwirtschaft für die Beseitigung von mikroben Organismen, zum Wäsche waschen, in Anwendungen für Schwimmbäder oder sogar als Prophylaxe gegen Fußpilz. Bedarfsweise und in geeigneter Rezeptur kann derartiger Anolyt aber auch als Wirkstoff zur Bekämpfung von Mikroben oder Keimen jeglicher Art, insbesondere Bakterien, Viren, Funghi oder dergleichen, eingesetzt werden.

Unter anderem ist auch ein Einsatz eines derartigen Anolyten oder einer derartigen elektrochemisch aktivierten Salzlösung, die u. a. einen Gehalt an freiem Chlor von mehr als 300 mg/l aufweist, als Mittel zur Behandlung von Wunden, insbesondere zur Reinigung und Desinfektion von Wunden zur Beschleunigung des Heilungsprozesses, erstrebenswert. Hierzu ist die Bereitstellung des Anolyten oder der elektrochemisch aktivierten Salzlösung in einer Form wünschenswert, die einerseits eine vergleichsweise einfache Handhabung insbesondere beim Aufbringen auf die zu behandelnden Wunden ermöglicht, andererseits aber auch eine hohe mikrobiozide Wirksamkeit des Wirkstoffs bei besonders langer Haltbarkeit, also insbesondere hoher Langzeitstabilität, ermöglicht. Hierzu wird angestrebt, die elektrochemisch aktivierte Salzlösung, also den Anolyten, in Form eines Gels vorzuhalten, das neben den genannten Bedingungen besonders gute Lagerbeständigkeit und Langzeitstabilität insbesondere im Hinblick auf die mikrobiozide Wirksamkeit des Anolyten gewährleistet.

Der Erfindung liegt daher die Aufgabe zu Grunde, ein Gel, insbesondere zur Verwendung in einem Wundbehandlungsmittel oder als Wundbehandlungsmittel, anzugeben, das in besonderer Weise die genannten Erfordernisse erfüllt. Zudem soll ein für die Herstellung des Gels besonders geeignetes Verfahren angegeben werden.

Bezüglich des Gels wird diese Aufgabe erfindungsgemäß gelöst, indem das Gel ein anorganisches Silikat als Verdicker sowie eine elektrochemisch aktivierte wässrige Kochsalzlösung umfasst, wobei die elektrochemisch aktivierte wässrige Kochsalzlösung, die einen Gehalt an freiem Chlor von mehr als 200 mg/l aufweist, wobei die elektrochemisch aktivierte wässrige Kochsalzlösung eine Leitfähigkeit von höchstens 12 mS/cm aufweist.

Vorteilhafte Ausgestaltungen der Erfindung sind Gegenstand der Unteransprüche.

Wie sich überraschend herausgestellt hat, ist unter der Vielzahl möglicher Verdicker oder Gelbildner die gewünschte Kombination der Eigenschaften für das Gel erreichbar, indem ein anorganisches Silikat als Verdicker ausgewählt wird. Durch die Auswahl eines derartigen Materials als Verdicker können insbesondere die ansonsten erwünschten Eigenschaften der elektrochemisch aktivierten Kochsalzlösung, insbesondere die mikrobiozide Wirksamkeit, auch nach dem Gelbildungsprozess erhalten werden.

Wie sich überraschenderweise herausgestellt hat, ist für die Einhaltung der genannten Erfordernisse, also Erhaltung der hohen mikrobioziden Wirksamkeit des Anolyten oder der elektrochemisch aktivierten Kochsalzlösung, auch über lange Lagerzeiträume hinweg, also für eine hohe Langzeit-Stabilität, die elektrische Leitfähigkeit der elektrochemisch aktivierten wässrigen Kochsalzlösung ein besonders bedeutsamer Parameter. Insbesondere ist dabei bei der Herstellung eines Gels die gewünschte hohe Langzeitstabilität erreichbar, indem eine elektrische Leitfähigkeit der elektrochemisch aktivierten wässrigen Kochsalzlösung von höchstens 12 mS/cm eingestellt wird. Grundsätzlich kann dazu die elektrochemisch aktivierte Kochsalzlösung geeignet, also mit entsprechend gewählter Leitfähigkeit, hergestellt werden. Alternativ oder in bevorzugter Ausgestaltung kann die elektrochemisch aktivierte Kochsalzlösung aber auch durch geeignete Vermischung oder Verdünnung einer elektrochemisch aktivierten Ausgangs-Kochsalzlösung (Anolyt) mit destilliertem Wasser bereitgestellt werden. Die elektrochemisch aktivierte wässrige Kochsalzlösung ist somit erhältlich durch Vermischen von elektrochemisch aktivierter Ausgangs-Kochsalzlösung, die einen Gehalt an freiem Chlor von mehr als 300 mg/l aufweist, mit destilliertem Wasser. Besonders bevorzugt wird dabei als Ausgangs-Kochsalzlösung der Anolyt oder die elektrochemisch aktivierte Salzlösung verwendet, wie sie mit dem in der EP 10 003 555.9 beschriebenen Verfahren erhältlich ist. Vorzugsweise wird als Ausgangs-Kochsalzlösung somit "Aseca-Lösung" verwendet.

Um die angestrebte hochgradig mikrobiozide Wirksamkeit des Gels zu gewährleisten, weist die Ausgangs-Kochsalzlösung, also besonders bevorzugt der genannte Anolit oder insbesondere die "Aseca-Lösung", vorteilhafterweise einen Gehalt an freiem Chlor von mehr als 500 mg/l, besonders bevorzugt von mehr als 600 mg/l, und/oder ein Redoxpotenzial zwischen 600 und 100 mV, besonders bevorzugt zwischen 700 und 900 mV, insbesondere von etwa 800 mV, auf. In alternativer oder zusätzlicher vorteilhafter Ausgestaltung weist die Ausgangs-Kochsalzlösung, also besonders bevorzugt der Anolyt oder die "Aseca-Lösung", eine Leitfähigkeit zwischen 12 und 16 mS/cm, besonders vorteilhaft von etwa 14 mS/cm und/oder einen pH-Wert zwischen 6 und 8, besonders bevorzugt von etwa 7, auf.

Wie sich überraschend herausgestellt hat, ist für den Verdickungs- oder Gelbildungsprozess, insbesondere in Kombination mit der vorgesehenen Verwendung eines anorgangischen Silikats als Verdicker, die Leitfähigkeit der durch Verdünnen oder Vermischen der elektrochemisch aktivierten Ausgangs-Kochsalzlösung (insbesondere Anolyt oder "Aseca-Lösung") mit destilliertem Wasser erhaltenen elektrochemisch aktivierten wässrigen Kochsalz-Lösung von besonderer Bedeutung. Erfindungsgemäß ist daher vorgesehen, die elektrische Leitfähigkeit der durch Vermischung oder Verdünnung der Ausgangs-Kochsalzlösung mit destilliertem Wasser erhältichen wässrigen Kochsalz-Lösung durch geeignete Wahl der Mischungs- oder Verdünnungsverhältnisse auf einen zur Begünstigung des Verdickungs- oder Gelbildungsprozesses besonders geeigneten Wert einzustellen. Hierzu ist beim bevorzugten Rückgriff auf Anolyt oder "Aseca-Lösung", wie sie nach dem in der EP 10 003 555.9 beschriebenen Verfahren erhältlich ist, eine elektrische Leitfähigkeit der verdünnten, also mit destiliertem Wasser vermischten, Kochsalzlösung von zwischen 10 und 12 mS/cm besonders bevorzugt von etwa 11 mS/cm, besonders vorteilhaft.

Das zur Verwendung als Verdicker oder Gelbildner vorgesehene anorganische Silikat ist, gerade in Kombination mit dem besonders bevorzugt gewählten Anolyt ("Aseca-Lösung"), in vorteilhafter Ausgestaltung ein Lithium-Magnesium-Natrium-Silikat, besonders vorteilhaft aus der Montmorillonit-Gruppe, ganz besonders vorteilhaft Lucentite-SWN. Das ganz besonders bevorzugt vorgesehene Lucentite-SWN ist als Lithium-Magnesium-Natrium-Silikat beispielsweise unter den CAS-Nummern 53320-86-8, 149316-65-4 oder 1217347 oder unter EINECS-Nr.258-476-2 erhältlich. Lucentite-SWN ist ein Lithium-Magnesium-Natrium-Silikat der Montmorillonit-Gruppe und somit ein Schicht-Silikat und entspricht im Wesentlichen auch dem Lucentite SAN, ohne dass aber quaternium-18 zugegeben ist.

Dieser Gelbildner ist insbesondere wegen seines Quellvermögens bei geringen Scherkräften besonders bevorzugt und vorliegend gerade für eine Kombination mit der elektrochemisch aktivierten Kochsalzlösung besonders geeignet, da es nicht weiter oxidierbar ist und somit den Erhalt der Wirksamkeit und Reaktivität der Kochsalzlösung nicht beeinträchtigt. Zudem ist es üblicherweise synthetisch hergestellt und daher auf Grund seiner Freiheit von Verunreinigungen besonders effizient. Andere Wasser aufnehmende Gelbildner, insbesondere solche aus der Montmorillonit-Gruppe, können aber ebenso geeignet sein. Eine weiterführende Erörterung von Lucentite-SWN und des verwandten Lucentite-SAN ist beispielsweise entnehmbar aus der US 6,660,277 B1 sowie der darin erwähnten US-amerikanischen Patentanmeldung Nr. 08/853,992.

Zur Herstellung des Gels, insbesondere unter Verwendung der vorstehend genannten wässrigen Kochsalzlösung in Kombination mit Lucentite-SWN als Verdicker oder Gelbildner, kann das Mischungsverhältnis Verdicker/Kochsalzlösung entsprechend dem gewünschten Einsatzzweck, also der gewünschten Viskosität, geeignet gewählt werden. Für vergleichsweise dickflüssige Anwendungen, die nicht tropfen sollen, wird vorzugsweise ein Mischungsverhältnis Verdicker/Kochsalzlösung zwischen etwa 3 und etwa 5 Gewichtsprozent (Verdickeranteil), besonders bevorzugt von etwa 4 Gewichtsprozent, gewählt. Für dünnflüssige Gele, die beispielsweise in eine Wunde oder dergleichen einfließen sollen, kann vorzugsweise ein Mischungsverhältnis zwischen 2 und 4 Gewichtsprozent (Verdicker) gewählt werden. Für besonders dünnflüssige bevorzugte Anwendungen, beispielsweise für zerstäubte Mittel oder nicht leicht abtropfende Pflanzenschutzmittel, wird hingegen vorzugsweise ein Mischungsverhältnis zwischen 0,1 und 2 Gewichtsprozent (Verdickeranteil) gewählt. Besonders bevorzugt wird das Gel unter Verwendung der genannten Bestandteile derart hergestellt, dass sich eine besonders gute Handhabbarkeit für den vorgesehenen Einsatzzweck, also insbesondere als Wundbehandlungsmittel oder als Bestandteil eines Wundbehandlungsmittels, ergibt. Dazu ist vorteilhafterweise u. a. vorgesehen, das Gel derart herzustellen, dass es eine Viskosität zwischen 4500 und 6500 mPas, besonders bevorzugt von etwa 5500 mPas, aufweist.

Wie sich zudem überraschenderweise herausgestellt hat, ist gerade für eine hohe Langzeitstabilität und Lagerbeständigkeit des hergestellten Gels unter Erhalt der hohen mikrobioziden Wirksamkeit der Ausgangs-Kochsalzlösung, insbesondere des bevorzugt verwendeten Anolyten oder der "ASECA-Lösung", der pH-Wert des hergestellten Gels von besonderer Bedeutung. Erfindungsgemäß ist daher vorgesehen, im Hinblick auf die verwendeten Bestandteile den pH-Wert des Gels geeignet einzustellen. Für die besonders bevorzugt vorgesehenen Bestandteile, nämlich insbesondere Anolyt oder "ASECA-Lösung" und geeignete Verdünnung mit destilliertem Wasser zur Bereitstellung der wässrigen Kochsalzlösung, in Verbindung mit Lucentite -SWN als Verdicker oder Gelbildner, ist für eine besonders hohe Langzeitstabilität ein pH-Wert von mindestens 9, besonders bevorzugt von etwa 10, besonders geeignet. Vorteilhafterweise wird bei der Herstellung des Gels daher insbesondere durch geeignete Wahl des Mischungsverhältnisses Verdicker/Kochsalzlösung ein pH-Wert von etwa 10 für das Gel eingestellt.

Bezüglich des Verfahrens wird die genannte Aufgabe gelöst, indem elektrochemisch aktivierte Ausgangs-Kochsalzlösung, die einen Gehalt an Chlor von mehr als 300 mg/l aufweist, unter Überwachung der elektrischen Leitfähigkeit durch Zudosierung derart mit destilliertem Wasser gemischt, dass die elektrische Leitfähigkeit der durch die Mischung entstehenden elektrochemisch aktivierten wässrigen Kochsalzlösung auf einen Wert von weniger als 12 mS/cm, vorzugsweise auf einen Wert von etwa 11 mS/cm, absinkt, wobei der wässrigen Kochsalzlösung anschließend ein anorganisches Silikat als Verdicker zugemischt wird.

Vorzugsweise wird dabei als anorganisches Silikat ein Lithium-Magnesium-Natrium-Silikat, vorzugsweise ein Silikat aus der Montmorillonit-Gruppe, besonders bevorzugt Lucentite-SWN, zugemischt.

In besonders vorteilhafter Ausgestaltung und insbesondere um die gewünschte hohe Langzeitstabilität und Lagerfähigkeit des Gels unter Aufrechterhaltung einer hohen mikrobioziden Wirksamkeit zu gewährleisten, wird der Verdicker oder der Gelbildner dabei derart zugemischt, dass das entstehende Gel einen pH-Wert von mindestens 9, vorzugsweise von zwischen 9 und 10, aufweist.

Die mit der Erfindung erzielten Vorteile bestehen insbesondere darin, dass basierend auf einer elektrochemisch aktivierten Ausgangs-Kochsalzlösung mit einem Gehalt an freiem Chlor von mehr als 300 mg/l durch Verdünnung der Ausgangs-Kochsalzlösung in destilliertem Wasser derart, dass die entstehende wässrige Kochsalz-Lösung eine elektrische Leitfähigkeit von höchstens 12 mS/cm aufweist, und durch Zugabe eines anorganischen Silikats als Verdicker oder Gelbildner zuverlässig ein für die Verwendung als Wundbehandlungsmittel geeignetes Gel mit besonders hoher mikrobiozider Wirkung bereitgestellt werden kann. Gerade durch die besonders bevorzugt vorgesehene Einstellung des pH-Werts des Gels auf einen Wert von etwa 10, insbesondere durch geeignete Wahl der Mischungsverhältnisse, kann dabei eine besonders hohe Langzeitbeständigkeit und Lagerfähigkeit des Gels ohne übermäßigen unerwünschten Verlust an mikrobiozider Wirksamkeit erreicht werden. Gerade in der Kombination mit Lucentite-SWN kann dabei ein Gel bereitgestellt werden, das bei Erhalt der mikrobioziden Wirksamkeit lager- und formstabil ist und seine Konsistenz behält, ohne zum Zerlaufen oder Zerfließen zu neigen.

Ein Ausführungsbeispiel der Erfindung, insbesondere betreffend die Herstellung des Gels, wird nachfolgend näher erläutert.

Als Basisstoff oder Ausgangs-Kochsalzlösung wird im vorliegenden Ausführungsbeispiel die elektrochemisch aktivierte Salzlösung verwendet, die durch Herstellung nach dem in der nicht vorveröffentlichten EP 10 003 555.9 beschriebenen Verfahren in der dort offenbarten Anlage erhältlich ist, und die als Anolyt oder als "ASECA-Lösung" bezeichnet wird. Ein derartiger Anolyt oder "ASECA-Lösung" weist einen Gehalt an freiem Chlor von etwa 700 mg/l, ein Redoxpotential von etwa 800 mV, einen pH-Wert von etwa 7 und eine elektrische Leitfähigkeit von etwa 14 mS/cm auf. Ein derartiger Anolyt oder "ASECA-Lösung" wird gemäß dem in der EP 10 003 555.9 beschriebenen Verfahren elektrolytisch und durch elektrochemische Aktivierung einer Kochsalzlösung hergestellt. Alternativ könnte als Ausgangsstoff aber natürlich auch eine anderweitig hergestellte geeignete Kochsalzlösung verwendet werden.

Diese elektrochemisch aktivierte Ausgangs-Kochsalzlösung wird durch Vermischung mit destilliertem Wasser verdünnt und mit Verdicker versehen. Grundsätzlich könnte dabei zunächst ein Vorprodukt aus Verdicker und destilliertem Wasser hergestellt werden, in das dann die elektrochemisch aktivierte Ausgangs-Kochsalzlösung, also vorliegend Anolyt oder "ASECA-Lösung", eingearbeitet wird. Bevorzugt ist aber, wie nachfolgend beschrieben, dass zunächst die elektrochemisch aktivierte Ausgangs-Kochsalzlösung mit destilliertem Wasser verdünnt und erst anschließend der Verdicker oder Gelbildner zugegeben wird.

Dazu wird die elektrochemisch aktivierte Ausgangs-Kochsalzlösung, also Anolyt oder "ASECA-Lösung", durch Zumischung von destilliertem Wasser verdünnt. Die Verdünnung erfolgt dabei durch dosierte Zugabe von destilliertem Wasser, wobei als geeigneter Parameter zur Prozessführung die elektrische Leitfähigkeit der verdünnten, wässrigen Kochsalz-Lösung gemessen wird. Die Zugabe von destilliertem Wasser, d. h. die Verdünnung, wird dabei derart und solange vorgenommen, bis die Leitfähigkeit der Mischung, also der verdünnten, wässrigen Kochsalzlösung, auf einen Wert von etwa 11 mS/cm abgesenkt ist.

Der solchermaßen vorbereiten wässrigen Kochsalzlösung wird anschließend Lucentite-SWN, also ein anorganisches Silikat, als Verdicker oder Gelbildner beigemischt. Die Zugabe des Verdickers erfolgt dabei etwa in einem Mischungsverhältnis von Verdicker/Kochsalzlösung von etwa 4 %, wobei als Parameter der Prozessführung u. a. die Viskosität des entstehenden Gels ermittelt wird. Diese wird vorteilhaft auf einen Zielwert von etwa 5500 mPa eingestellt. Des Weiteren wird als gerade für die Langzeitstabilität und Lagerfähigkeit des entstehenden Gels bedeutsamer Parameter der pH-Wert des Gels ermittelt. Die Zuspeisung des Verdickers erfolgt dabei derart, dass das entstehende Gel einen pH-Wert von mindestens 9, vorzugsweise von etwa 10, aufweist.

Die Zugabe des Verdickers oder Gelbildners erfolgt dabei durch sukzessives Einstreuen in die Mischung unter Rühren. Anschließend erfolgt zunächst unter weiterem Rühren ein Vorquellen und sodann unter Erhöhung der Rührgeschwindigkeit ein Homogenisieren.

## Patentansprüche

1. Gel, insbesondere zur Verwendung in einem Wundbehandlungsmittel, umfassend ein anorganisches Silikat als Verdicker und eine elektrochemisch aktivierte wässrige Kochsalzlösung, die einen Gehalt an freiem Chlor von mehr als 200 mg/l aufweist, wobei die elektrochemisch aktivierte wässrige Kochsalzlösung eine Leitfähigkeit von höchstens 12 mS/cm aufweist.

2. Gel nach Anspruch 1, wobei die elektrochemisch aktivierte wässrige Kochsalzlösung erhältlich ist durch Vermischen von elektrochemisch aktivierter Ausgangs-Kochsalzlösung, die einen Gehalt an freiem Chlor von mehr als 300 mg/l aufweist, mit destilliertem Wasser.

3. Gel nach Anspruch 1 oder 2, bei dem das Mischungsverhältnis Verdicker/ Kochsalzlösung zwischen etwa 3 und etwa 5 Gew.% beträgt.

4. Gel nach Anspruch 2 oder 3, bei dem die Ausgangs-Kochsalzlösung einen Gehalt an freiem Chlor von mehr als 500 mg/l, vorzugsweise von mehr als 600 mg/l, aufweist.

5. Gel nach einem der Ansprüche 2 bis 4, bei dem die Ausgangs-Kochsalzlösung ein Redoxpotential zwischen 600 und 1000 mV, vorzugsweise zwischen 700 und 900 mV, besonders bevorzugt von etwa 800 mV, aufweist.

6. Gel nach einem der Ansprüche 2 bis 5, bei dem die Ausgangs-Kochsalzlösung eine Leitfähigkeit zwischen 12 und 16 mS/cm aufweist.

7. Gel nach einem der Ansprüche 2 bis 6, bei dem die Ausgangs-Kochsalzlösung einen pH-Wert zwischen 6 und 8, vorzugsweise von etwa 7, aufweist.

8. Gel nach einem der Ansprüche 1 bis 7, bei dem die Kochsalzlösung eine Leitfähigkeit zwischen 10 und 12 mS/cm, vorzugsweise von etwa 11 mS/cm, aufweist.

9. Gel nach einem der Ansprüche 1 bis 8, bei dem als Verdicker ein Lithium-Magnesium-Natrium-Silikat, vorzugsweise ein Silikat aus der Montmorillonit-Gruppe, besonders bevorzugt Lucentite-SWN, vorgesehen ist.

10. Gel nach einem der Ansprüche 1 bis 9, das einen pH-Wert von mindestens 9, vorzugsweise von zwischen 9 und 10, und/oder eine Viskosität von zwischen 4500 und 6500 mPa, vorzugsweise von etwa 5500 mPa, aufweist.

11. Verfahren zur Herstellung eines Gels nach einem der Ansprüche 1 bis 10, bei dem elektrochemisch aktivierte Ausgangs-Kochsalzlösung, die einen Gehalt an freiem Chlor von mehr als 300 mg/l aufweist, unter Überwachung der elektrischen Leitfähigkeit durch Zudosierung derart mit destilliertem Wasser gemischt wird, dass die elektrische Leitfähigkeit der durch die Mischung entstehenden elektrochemisch aktivierten wässrigen Kochsalzlösung auf einen Wert von weniger als 12 mS/cm, vorzugsweise auf einen Wert von etwa 11 mS/cm, absinkt, und bei dem der wässrigen Kochsalzlösung anschließend ein anorganisches Silikat als Verdicker zugemischt wird.

12. Verfahren nach Anspruch 11, bei dem als anorganisches Silikat ein Lithium-Magnesium-Natrium-Silikat, vorzugsweise ein Silikat aus der Montmorillonit-Gruppe, besonders bevorzugt Lucentite-SWN, zugemischt wird.

13. Verfahren nach Anspruch 11 oder 12, bei dem der Verdicker derart zugemischt wird, dass das entstehende Gel einen pH-Wert von mindestens 9, vorzugsweise von zwischen 9 und 10, aufweist.
